# EUROPEAN PATENT APPLICATION

(11) **EP 4 040 150 A1**
(43) Date of publication of application: **10.08.2022**
(21) Application number: 21305146.9
(22) Date of filing: 04.02.2021
(51) Int. Cl.: G01N 33/18

(54) **METHOD FOR THE DETECTION OF POLYCYCLIC AROMATIC HYDROCARBONS**

(71) Applicant: Centre national de la recherche scientifique, 75016 Paris (FR); Université Grenoble Alpes, 38400 Saint-Martin-d'Hères (FR)
(72) Inventor: LE GOFF, Alan, 38830 Crêts-en-Belledonne (FR); SORRENTINO, Ilaria, 06000 Nice (FR)
(74) Representative: Lavoix

(57) **Abstract**

The present invention relates to a method for the detection of polycyclic aromatic hydrocarbons comprising:
a) a step of contacting an aqueous solution comprising at least one polycyclic aromatic hydrocarbon with a catalyst for the oxidation of polycyclic aromatic hydrocarbons and with an electrode made of a conductive porous material, and
b) a step of detecting the anthraquinone that is formed during the previous step through the oxidation of the polycyclic aromatic hydrocarbons.

## Description

The present invention concerns a method for the detection of polycyclic aromatic hydrocarbons, as well as a kit for the detection of said polycyclic aromatic hydrocarbons and a biosensor for polycyclic aromatic hydrocarbons.

Polycyclic aromatic hydrocarbons (PAHs) are among the most common organic pollutants found at contaminated industrial sites around the world. Many PAHs can have detrimental effect on the flora and fauna of affected habitats through uptake and accumulation in food chains, and in some instances, pose serious health problems and/or genetic defects in humans. PAHs, such as pyrene, benzo[a]pyrene and benz[a]anthracene have toxic, mutagenic and/or carcinogenic properties (Mastragela G, Fadda E, Marzia V. Polycyclic aromatic hydrocarbons and cancer in man. Environ Health Perspect 1996;104:1166-70; and Goldman R, Enewold L, Pellizzari E, Beach JB, Bowman ED, Krishman SS, et al. Smoking increase carcinogenic polycyclic aromatic hydrocarbons in human lung tissue. Cancer Res 2001 ;61:6367-71.).

Currently, the US. Environmental Protection Agency has classified 16 PAHs as priority pollutants whose remediation is considered indispensable for environmental clean up and human health (Liu K, Han W, Pan WP, Riley JT. Polycyclic aromatic hydrocarbon (PAH) emissions from a coal fired pilot FBC system. J Hazard Mater 2001 ;84:175-88).

Current methods for PAH sensing are based on gaz or liquid chromatography coupled to mass spectrometry, fluorimetry or UV-visible spectroscopy. These techniques use heavy laboratory instrumentation and/or expensive light sources.

There is thus a need for a method for PAH sensing being easy to be implemented and being also inexpensive.

The aim of the present invention is to provide a method for the detection of PAH, said method being sensitive, fast and cheap.

Another aim of the present invention is to provide a method for the detection of PAH, said method being easily implemented and not requiring a preliminary treatment of the sample wherein the PAH is to be detected.

Therefore, the present invention relates to a method for the detection of polycyclic aromatic hydrocarbons comprising:
a) a step of contacting an aqueous solution comprising at least one polycyclic aromatic hydrocarbon with a catalyst for the oxidation of polycyclic aromatic hydrocarbons and with an electrode made of a conductive porous material, and
b) a step of detecting the anthraquinone that is formed during the previous step through the oxidation of the polycyclic aromatic hydrocarbons.

The method of the present invention thus consists in using a catalyst for the oxidation of PAH in the sample wherein the PAH is/are to be detected, said sample being in the form of an aqueous solution.

The present invention is based on the surprising interaction between the product obtained by the oxidation of the PAH and the conductive porous material. Indeed, it has been surprisingly observed by the inventors that the anthraquinone obtained after step a) has high adsorption properties for its interaction with the conductive porous material of the electrode.

Step a) of the method of the invention thus involves the use of a catalyst being able to generate quinones or semi-quinones on polycyclic rings.

Said catalyst is useful for the oxidation of the PAH(s) present in the sample wherein the PAH is/are to be detected, leading then to the formation of anthraquinone, which is the product of said oxidation.

According to an embodiment, the catalyst for the oxidation of polycyclic aromatic hydrocarbons is selected from the group consisting of:
- laccases and related enzymes such as copper oxidases, tyrosinases, and peroxidases);
- electrocatalysts of PAH oxidation, such as metals chosen from: Au, Pt, Ti, Ag, Pd, W, Mo, Fe, Zn, Cu or Mo and metallic alloys, metallic nanoparticles and oxides and carbon-based materials (for example charcoal, activated carbon, carbon black, carbon nanotubes and fibers, graphene);
- molecular catalysts such as iron complexes (for example porphyrins and iron chlorides), ruthenium complexes (ruthenium chloride), nickel complexes (nickel pyrazoyl), titanium complexes, vanadium complexes, or copper complexes; and
- polyoxometallates based on ruthenium, vanadium, tungsten or molybdenum.

Preferably, the catalyst for the oxidation of polycyclic aromatic hydrocarbons is a laccase.

The laccases (EC 1.10.3.2) are a family of enzymes which are found in many plants, fungi and microorganisms.

*In vivo,* laccases have an oxidizing activity and act as a catalyst within an enzymatic oxidation process.

The laccases which may be used in the method of the invention may be derived from plants, from fungi or microorganisms. The laccases stemming from fungi notably include the laccases of the *Aspergillus, Neurospora* (for example *Crassa Neurospora), Podospora, Botrytis, Collybia, Fomes, Lentinus, Pleurotus, Trametes* (for example *Trametes villosa* and *Trametes versicolor*), *Rhizoctonia* (for example *Rhizoctonia solani*)*, Coprinus* (for example *Coprinus cinereus, Coprinus comatus, Coprinus friesii* and *Coprinus plicatilis*)*, Psathyrella* (for example *Psathyrella condelleana*), *Panaeolus* (for example *Panaeolus papilionaceus*), *Myceliophthora* (for example *Myceliophthora thermophila*), *Schytalidium* (for example *Schytalidium thermophilum*), *Polyporus* (for example *Polyporus pinsitus*), *Phlebia* (for example *Radiata phlebia*), *Pycnoporus* (for example *Pycnoporus cinnabarinus*) or *Coriolus* (for example *Coriolus hirsutus*) genera. The laccases stemming from bacteria stem for example from *Bacillus.*

Preferably, a laccase stemming from *Trametes versicolor,* marketed by Sigma Aldrich is used.

Concentrations of catalyst used within the invention modify the conversion rate of the PAH into a redox-active detectable compound. Preferably, the catalyst concentrations, in particular the laccase concentrations, when used in solution, range between 1 and 1000 U mL⁻¹.

As mentioned above, the method of the present invention also involves the use of an electrode made of a conductive porous material.

According to the invention, the term "conductive material" refers to a material which allows the flow of electrons in one or more directions across its structure. Conductivity of these materials is given by its electrical conductivity in Siemens and its electrical resistivity in ohm.meter

According to the invention, the term "porous material" refers to a material comprising at least one pore, preferably several pores, said pore(s) having a size comprised from 1 nm to 1 mm. Porosity can be given by measuring the specific surface area of the material by gas physical adsorption giving access to the BET surface which can be typically comprised between 1 and 5000 m² g⁻¹.

According to an embodiment, the conductive porous material is selected from the group consisting of: carbon-based conductive material, conductive polymers and metals and corresponding metal oxides.

As carbon-based conductive material, the followings may be mentioned: carbon nanotubes, graphene, activated charcoal, carbon black, carbon pastes, and graphite.

As conductive polymers, the followings may be mentioned: polypyrroles, polythiophene, polyvinyl, polyacetylenes, polyindoles, polyanilines, polyphenylenes, and polypyrenes.

As metals, the followings may be mentioned: Au, Pt, Ti, Ag, Pd, W, Mo, Fe, Zn, Cu and Mo.

Preferably, the electrode made of a conductive porous material for step a) is an electrode made of carbon nanotubes. The electrode can be hard disks of 1 µm to 1 cm, screen-printed electrodes, flexible paper or bucky-paper electrodes, microelectrodes, microelectrode arrays, microneedles, nanoneedles, or Field-Effect-Transistor electrodes.

Step a) as defined above is then followed by a detection step, consisting in the detection of the anthraquinone that is formed during the previous step through the oxidation of the polycyclic aromatic hydrocarbons.

According to an embodiment, said catalyst is put in the presence of the solution to be analyzed which contains the targeted PAH (in particular anthracene). The catalyst triggers the oxidation of the PAH (anthracene) into a quinoid product which is redox-active (in particular anthraquinone). Said conductive material is put in the presence of the solution containing the oxidized product. The conductive material affords the adsorption of the product and its detection by electrochemistry.

As PAH able to be detected according to the present method, the following compounds may be mentioned: Naphtalene, Benzo(k)fluoranthene, Acenaphtene, Benzo(a)pyrene, Acenaphtylene, Dibenz(a,h)anthracene, Fluorene, Benzo(ghi)perylene, Phenanthrene, Indeno(1,2,3-cd)pyrene, anthracene, Fluoranthene, Pyrene, Benz(a)anthracene, Chrysene, Benzo(b)fluoranthene, perylene, and coronene.

According to a particular embodiment, step a) of the method according to the present invention further comprises the addition of a redox compound.

Preferably, the redox compound is selected from the group consisting of: 2,2'-azino-bis(3-ethylbenzothiazoline-6-sulfonic acid)(ABTS), (2,2,6,6-tetramethyl-piperidin-1-yl)oxy (TEMPO), osmium complexes, quinones, metallocenes such as ferrocenes, and mixtures thereof.

The use of such redox compound is advantageous in that it facilitates the oxidation reaction and also in that the method of the invention is more sensitive.

Preferably, the concentration of the redox compound is between 1 nM and 500 mM.

According to an embodiment, the catalyst for the oxidation of polycyclic aromatic hydrocarbons as defined above is immobilized on the electrode.

In such embodiment, step a) thus consists in contacting the aqueous solution (or sample) comprising at least one polycyclic aromatic hydrocarbon with the electrode (on which is immobilized the catalyst as defined above).

This specific embodiment has the advantage to be even more simple by combining both the catalyst and the electrode material in the same surface for a rapid detection method.

The present invention also relates to a kit for the detection of polycyclic aromatic hydrocarbons comprising:
- an electrode made of a conductive porous material as defined above, and
- a catalyst for the oxidation of polycyclic aromatic hydrocarbons as defined above.

Preferably, the kit according to the invention comprises an electrode made of carbon nanotubes and a laccase.

The present invention also relates to a biosensor for polycyclic aromatic hydrocarbons, said biosensor comprising an electrode made of a conductive porous material as defined above, on which a catalyst for the oxidation of polycyclic aromatic hydrocarbons as defined above is immobilized.

A preferred biosensor according to the present invention comprises an electrode made of carbon nanotubes, on which a laccase is immobilized.

### FIGURES

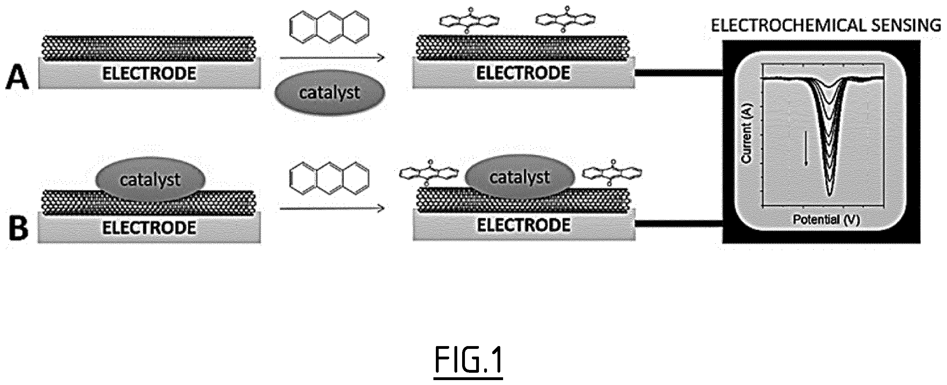

**Figure 1****.** Principle of the PAH sensor according to the invention based on (A) catalyst (such as laccase) in solution and (B) immobilized on the electrode (such as an electrode made of carbon nanotubes).

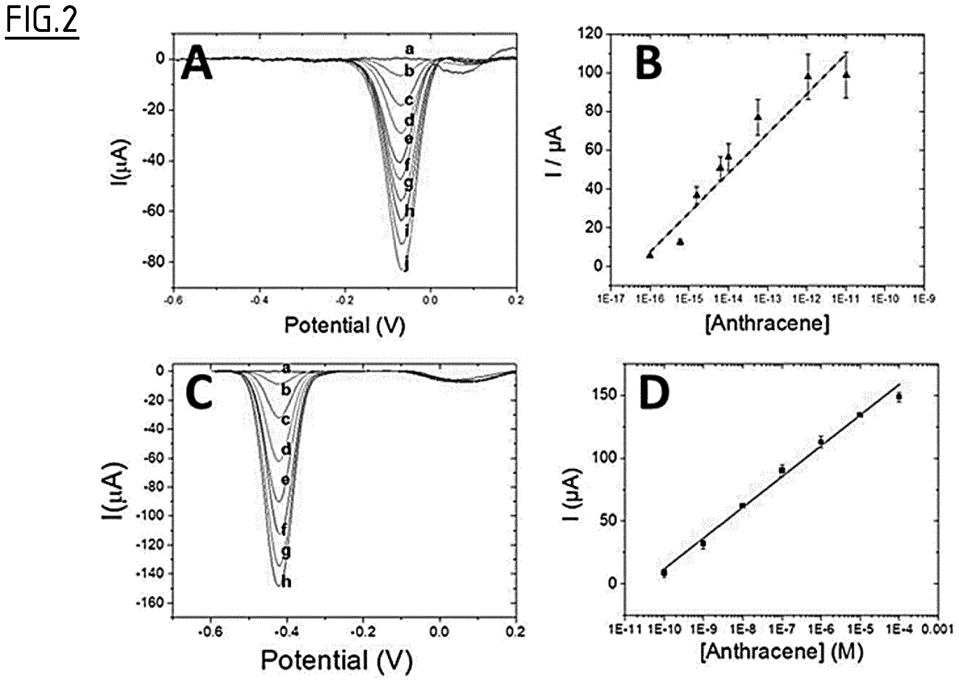

**Figure 2****.** (A) Electrochemical detection of different concentrations of anthracene solutions (a: 0, b: 0.1fM, c: 0.5fM, d: 1 fM, e: 10 fM, f: 50 fM, g: 0.1 pM, h:1 pM , i: 10 pM, and j: 50 pM) obtained with laccase in solution by SWV (0.1 M Mcllvaine buffer pH 5, 25°C, ref Ag/AgCI, pulse height = 25 mV, pulse width = 0.5 s, step height = -5 mV); (B) corresponding linear logarithmic scale between 0.1 fM and 50 pM; (C) Electrochemical detection of different concentrations of anthracene solutions (a: 0, b: 0.1 nM, c: 1 nM, d: 10 nM, e: 0.1 µM , f: 1 µM, g: 10 µM and h:100 µM) obtained with immobilized laccase by SWV (0.1 M Mcllvaine buffer pH 5, 25°C, ref Ag/AgCl, pulse height = 25 mV, pulse width = 0.5 s, step height = -5 mV); and (D) corresponding linear logarithmic scale between 0.1 nM and 0.1 µM.

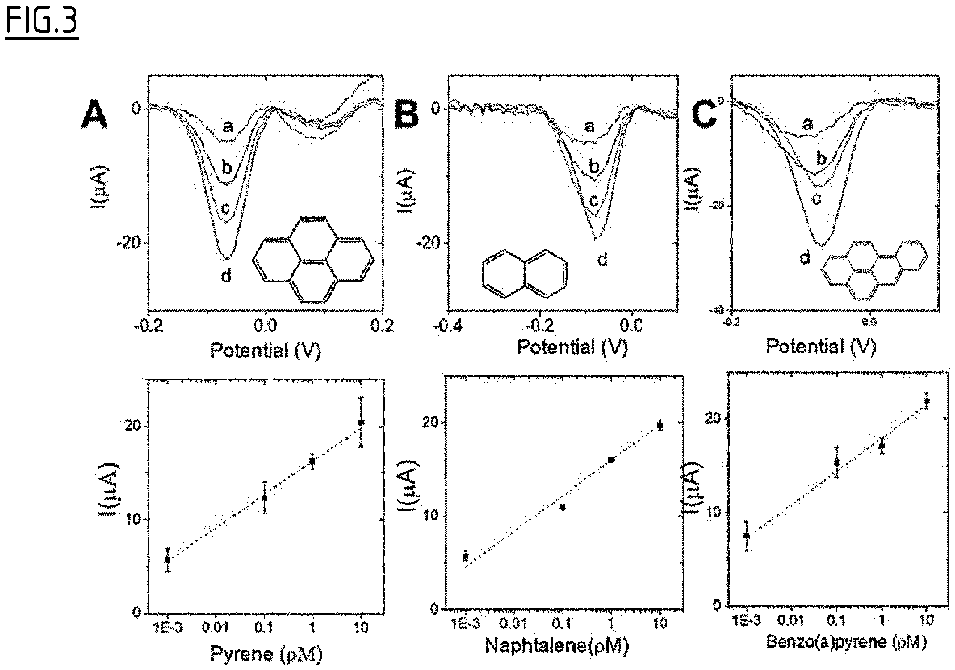

**Figure 3****.** (A) Electrochemical detection of different concentrations of pyrene, naphthalene and benzo(a)pyrene solutions (a: 0.001, b: 0.1 c:1, and d: 10 pM) obtained with laccase in solution by SWV (0.1 M Mcllvaine buffer pH 5, 25°C, ref Ag/AgCl, pulse height = 25 mV, pulse width = 0.5 s, step height = -5 mV) and (B) corresponding linear logarithmic scale between 1 fM et 10 pM.

### EXEMPLES

### Materials and Methods

### Reagents

All reagents were purchased from Sigma-Aldrich (Saint Louis, Missouri, USA) and were used without further purification. All chemicals employed were of analytical grade. Distilled water was passed through a Milli-Q water purification system to obtain 18.2 MΩ cm⁻¹ ultrapure water. Phosphate/citrate (Mcilvaine) and Tris-HCI buffer solutions ware prepared from Milli-Q water.

### Electrochemical Measurements

The electrochemical experiments were carried out in a three-electrode electrochemical cell using a Biologic VMP3 Multi Potentiostat. The saturated calomel electrode (SCE) served as the reference electrode, a Pt wire was used as the counter electrode and MWCNT bioelectrodes were used as working electrodes. All experiments were conducted at room temperature. All simulated curves were obtained via Origin Pro 9.0. Error bars were estimated from three measurements recorded per sample.

### Preparation of the glassy carbon- modified MWCNT electrode

The working electrodes were glassy carbon electrodes (3 mm diameter). 5 mg/mL NMP dispersions of MWCNTs (Multi-Walled Carbon Nanotube, purity > 99% Sigma-Aldrich) were prepared by 30 min in ultrasonic bath (Fisher scientific FB 15050) until homogeneous black suspension was obtained. Then 20 µL of the MWCNTs solution were drop-casted on a GCE and NMP was removed under vacuum obtaining a 5-µm-thick film.

### Laccase Enzymes

The laccase activity was assayed at room temperature, monitoring the oxidation of ABTS (2,2'-azino-bis(3-ethylbenzothiazoline-6-sulphonic acid)) at 420 nm (ε420 nm = 3.6 × 10 ⁴ M⁻¹ cm⁻¹)-the assay mixture contained 2 mM ABTS and 50 mM phosphate/citrate buffer, pH 3.0.

### PAH biosensing

Two different strategies have been set up:

### - Laccase in solution (Figure 2A and B, Figure 3)

A reaction solution was prepared with 2 U of laccase, 0.01 mM ABTS as a redox mediator in phosphate/citrate (2mL at pH= 5) at increasing PAH concentrations (from 0 to 1mM) and incubated at room temperature for 1 hour. MWCNT electrodes were immersed in the reaction solution for 10 minutes, and the detection of as-produced and adsorbed oxidized products were measured by SWV in a phosphate/citrate buffer solution pH 5.

### - 40°C/ no ABTS, immobilized enzyme (figure 2C and 2D)

In the second developed assay to detect PAHs, the modified MWCNT electrodes were incubated in 20 µL of laccase (100U/mL) for 2h at room temperature. Electrodes were then rinsed with 50mM Tris-HCI buffer solution at pH = 8 and stored at 4°C. The modified bioelectrodes were immersed in a phosphate/citrate buffer solution (2mL at pH= 5) at increasing concentrations of Anthracene (from 0 to 1 mM) and incubated at 40°C for 2h. Then, the detection of as-produced and adsorbed oxidized products were measured by SWV in a phosphate/citrate buffer solution pH 5.

## Claims

1. A method for the detection of polycyclic aromatic hydrocarbons comprising:
a) a step of contacting an aqueous solution comprising at least one polycyclic aromatic hydrocarbon with a catalyst for the oxidation of polycyclic aromatic hydrocarbons and with an electrode made of a conductive porous material, and
b) a step of detecting the anthraquinone that is formed during the previous step through the oxidation of the polycyclic aromatic hydrocarbons.

2. The method of claim 1, wherein the catalyst for the oxidation of polycyclic aromatic hydrocarbons is selected from the group consisting of: laccases and related enzymes, electrocatalysts of PAH oxidation, molecular catalysts, polyoxometallates based on ruthenium, vanadium, tungsten or molybdenum, and mixtures thereof.

3. The method of claim 1 or 2, wherein the catalyst for the oxidation of polycyclic aromatic hydrocarbons is a laccase.

4. The method of any one of claims 1 to 3, wherein the conductive porous material is selected from the group consisting of: carbon-based conductive materials, conductive polymers, and metals and corresponding metal oxides.

5. The method of any one of claims 1 to 4, wherein the electrode made of a conductive porous material is an electrode made of carbon nanotubes.

6. The method of any one of claims 1 to 5, wherein step a) further comprises the addition of a redox compound.

7. The method of claim 6, wherein the redox compound is selected from the group consisting of: 2,2'-azino-bis(3-ethylbenzothiazoline-6-sulfonic acid)(ABTS), (2,2,6,6-tetramethylpiperidin-1-yl)oxy (TEMPO), osmium complexes, quinones and metallocenes.

8. The method of any one of claims 1 to 7, wherein the catalyst for the oxidation of polycyclic aromatic hydrocarbons is immobilized on the electrode.

9. A kit for the detection of polycyclic aromatic hydrocarbons comprising:
- an electrode made of a conductive porous material as defined in any one of claims 1 to 8, and
- a catalyst for the oxidation of polycyclic aromatic hydrocarbons as defined in any one of claims 1 to 8.

10. A biosensor for polycyclic aromatic hydrocarbons comprising an electrode made of a conductive porous material as defined in any one of claims 1 to 8, on which a catalyst for the oxidation of polycyclic aromatic hydrocarbons as defined in any one of claims 1 to 8 is immobilized.
